# EUROPEAN PATENT APPLICATION

(11) **EP 1 512 365 A1**
(43) Date of publication of application: **09.03.2005**
(21) Application number: 03730651.1
(22) Date of filing: 28.05.2003
(51) Int. Cl.: A61B 1/04

(54) **ELECTRONIC ENDOSCOPE DEVICE**

(30) Priority: 31.05.2002 JP 2002160556
(71) Applicant: Olympus Corporation, Tokyo 151-0072 (JP)
(72) Inventor: SUZUKI, Tatsuhiko, Hino-shi, Tokyo 191-0052 (JP)
(74) Representative: von Hellfeld, Axel, Dr. Dipl.-Phys.
(86) International application number: PCT/JP2003/006629
(87) International publication number: WO 2003/101285

(57) **Abstract**

A CPU included in a video processor detects the type of CCD incorporated in an electronic endoscope actually connected to the video processor on the basis of identification information sent from an incorporated endoscope identification circuit. Moreover, when an optional board is loaded, an optional board detector detects an extension feature to be implemented on the optional board. For example, when the incorporated CCD offers a small number of pixels, restrictions are imposed for fear an enlargement/reduction circuit may perform enhancement based on electronic zooming. Moreover, an indication that the feature is invalid is established. Thus, a user is prevented from performing unnecessary handling. This results in improved maneuverability.

## Description

### Technical Field

The present invention relates to an electronic endoscope system in which an endoscopic image produced by an image pick-up means realized with a solid-state imaging device is displayed on a display means.

### Background Art

In recent years, electronic endoscope systems have widely prevailed as a modality that displays on a display device an endoscopic image, which is produced by an image pick-up device realized with a solid-state imaging device and is transmitted to the display device, so as to assist in endoscopic examination or diagnosis.

The electronic endoscope systems include a type of electronic endoscope system that permits use of electronic endoscopes whose solid-state imaging devices offer different numbers of pixels. Moreover, a related art concerning the electronic endoscope system, for example, the one described in Japanese Unexamined Patent Application Publication No. 2000-354240 provides a system including a signal processing unit in which an expansion board on which an extension feature such as a zooming feature is implemented can be loaded.

The publication describes that when the expansion board is loaded, a signal that has undergone extension processing is transmitted through an output terminal of the expansion board.

In the case of the related art described in the above publication, when the expansion board is loaded, whether the signal that has undergone extension processing is transmitted can be verified by checking if the output terminal of the expansion board is found. However, some electronic endoscope systems have an extension feature such as a zooming feature, though no expansion board is loaded. Whether the signal that has undergone extension processing is transmitted cannot always be verified by checking if an expansion board is loaded.

By the way, solid-state imaging devices are sorted into a plurality of types according to the different numbers of pixels. For example, when a solid-state imaging device adopted for an electronic endoscope system offers a small number of pixels, an extension feature such as a zooming feature should be preferably restricted in some cases. For example, although the number of pixels is small, if an image is enlarged based on electronic zooming, a coarse image may ensue. In short, enlargement should not be performed in some cases.

An object of the present invention is to provide an electronic endoscope system that restricts a usable processing feature according to an actual connection to a signal processing unit, and that enjoys improved maneuverability.

### Disclosure of Invention

An electronic endoscope system in accordance with a first aspect of the present invention comprises an electronic endoscope including a solid-state imaging device, and a signal processing unit that converts a signal read from the solid-state imaging device into a predetermined video signal. The electronic endoscope system further comprises a detector that detects the connection to the signal processing unit.

According to the first aspect of the present invention, a restricting unit is included for restricting processing to be performed by the signal processing unit according to the result of detecting performed by the detector. For example, the type of solid-state imaging device included in the electronic endoscope is detected. Depending on the detected type of solid-state imaging device, processing is restricted for fear unnecessary processing may be performed. In this case, presentation of the processing feature may be restricted so that the fact that the processing feature is invalid can be readily recognized. Otherwise, depending on whether an expansion board is present, the indication of a feature implemented on the expansion board may be restricted. Thus, ease of use is improved.

### Brief Description of the Drawings

Fig. 1 is a block diagram showing the overall configuration of an electronic endoscope system in accordance with a first embodiment of the present invention;
Fig. 2 is a block diagram showing the circuit elements of an enlargement/reduction circuit;
Fig. 3A and Fig. 3B present concrete examples of address data recorded in a lookup table;
Fig. 4A, Fig. 4B, and Fig. 4C present concrete examples of a mask size (screen image size) in which an endoscopic image is displayed;
Fig. 5A, Fig. 5B, and Fig. 5C show examples of an endoscopic image displayed in a semi-full mask size when enlargement is directed on the condition that an endoscopic image size should not be interlocked with a mask size;
Fig. 6 shows an example of a displayed menu screen image that helps a user determine various settings;
Fig. 7 shows an example of the layout of components on a front panel;
Fig. 8A, Fig. 8B, and Fig. 8C shows examples of an endoscopic image displayed when enlargement is directed on the condition that an endoscopic image size should be interlocked with a mask size;
Fig. 9 shows an example of a monitor screen image displayed when a CCD does not support electronic zooming feature;
Fig. 10 is a block diagram showing the circuit elements of a structure enhancement circuit;
Fig. 11 shows an example of display of an endoscopic image on which an image produced by a shape-of-endoscope detector is superimposed;
Fig. 12 is a flowchart describing initialization;
Fig. 13 is a flowchart describing major operations performed in the present embodiment when changing of mask sizes or enlargement is directed;
Fig. 14 is a block diagram showing the configuration of an extension processing unit and its peripherals employed in a second embodiment of the present invention; and
Fig. 15A and Fig. 15B are explanatory diagrams concerning structure enhancement to be achieved by adding dummy pixels to the border of an image.

### Best Mode for Carrying Out the Invention

Referring to the drawings, the embodiments of the present invention will be detailed below.

### (First Embodiment)

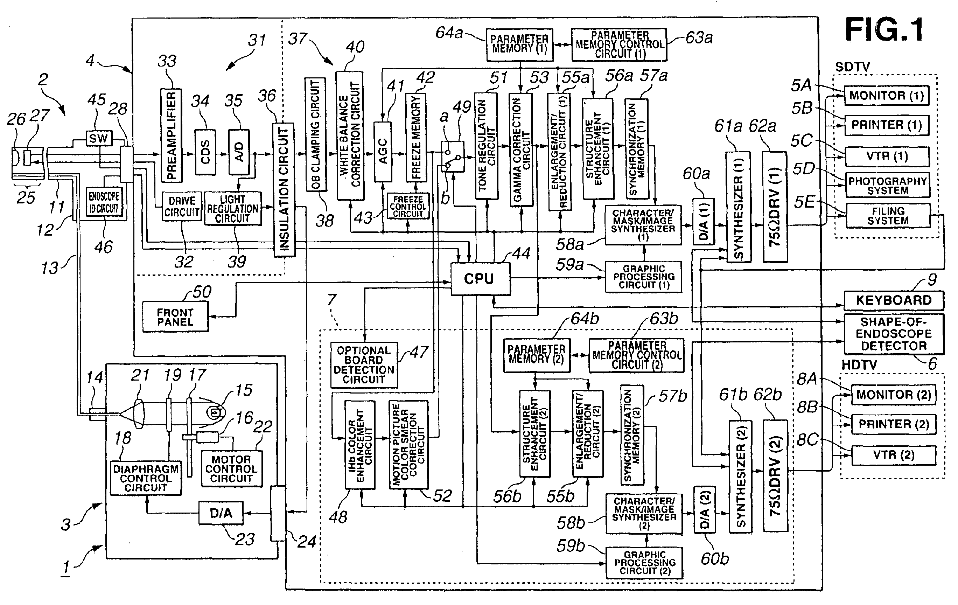

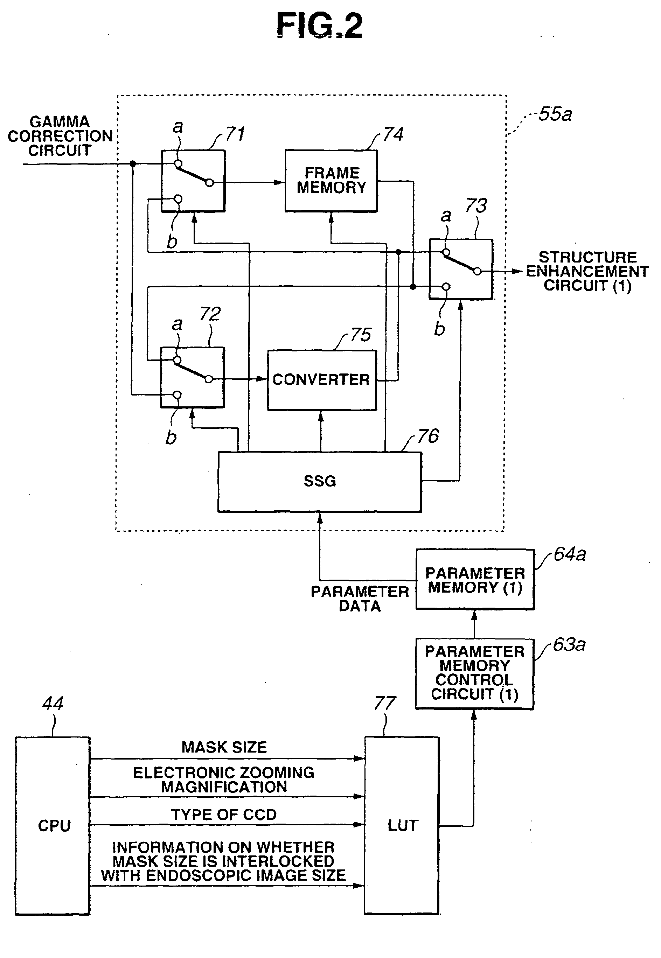

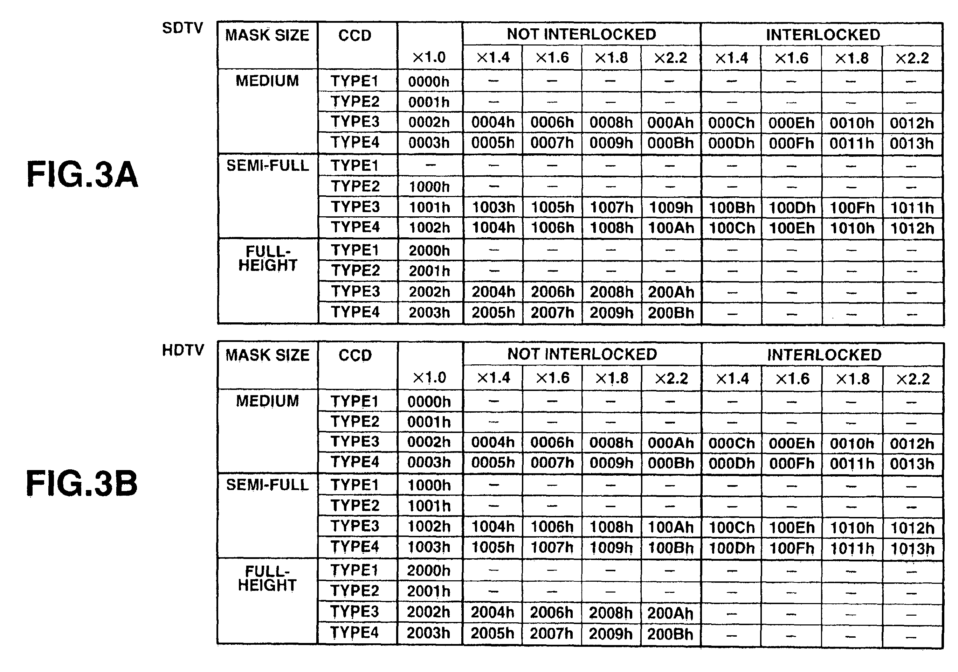

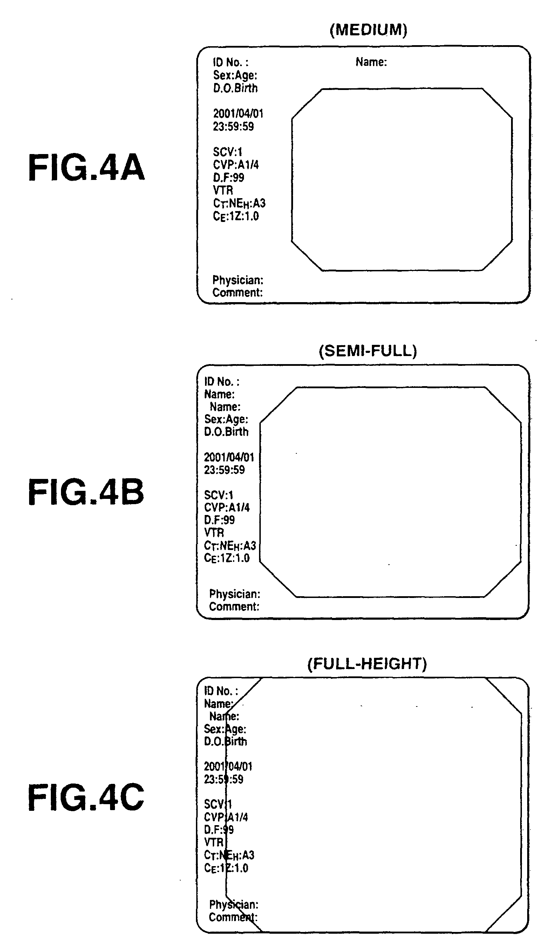

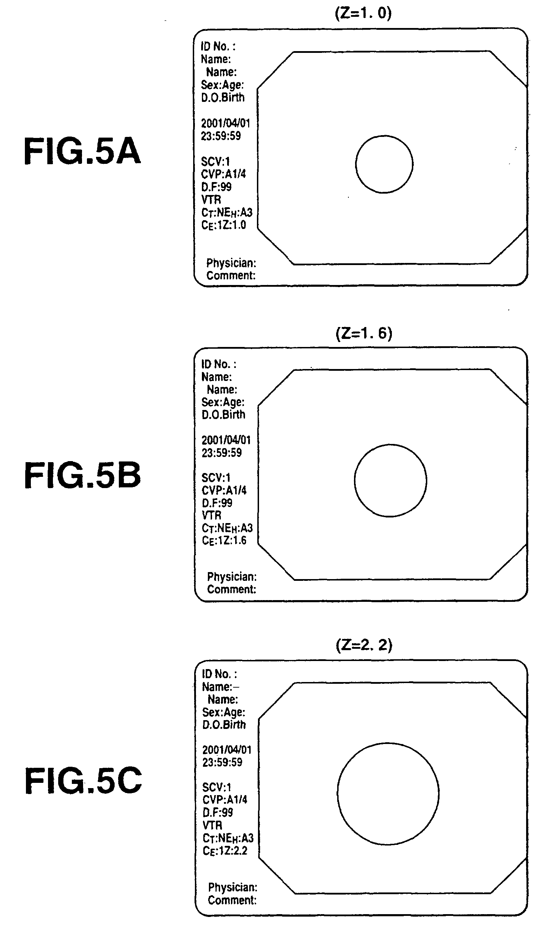

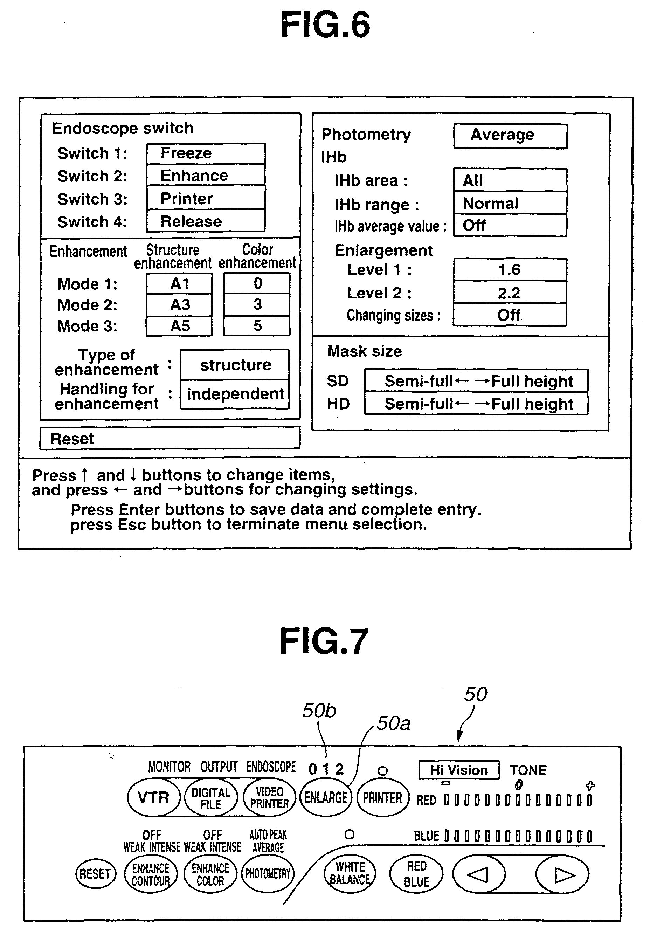

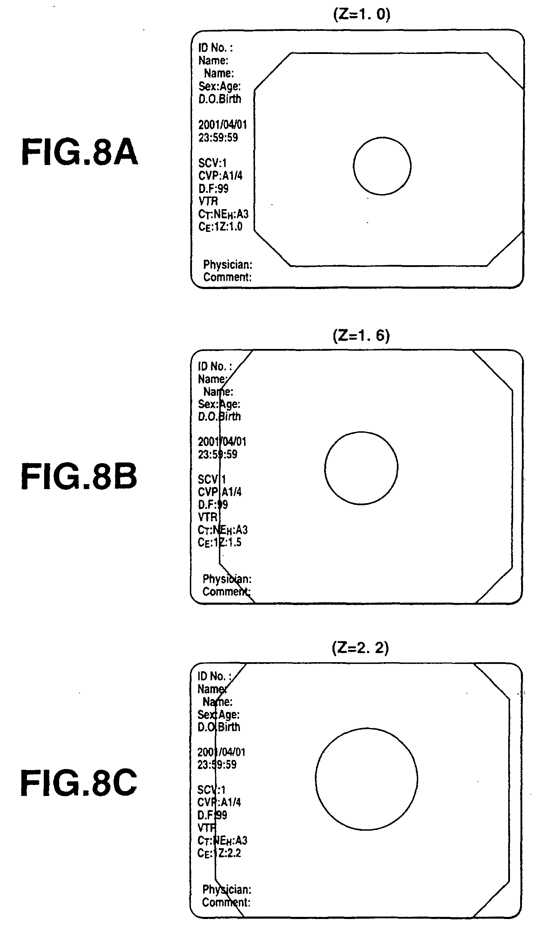

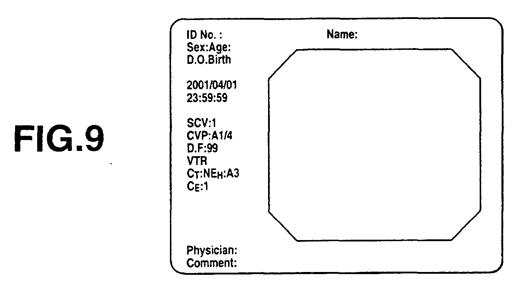

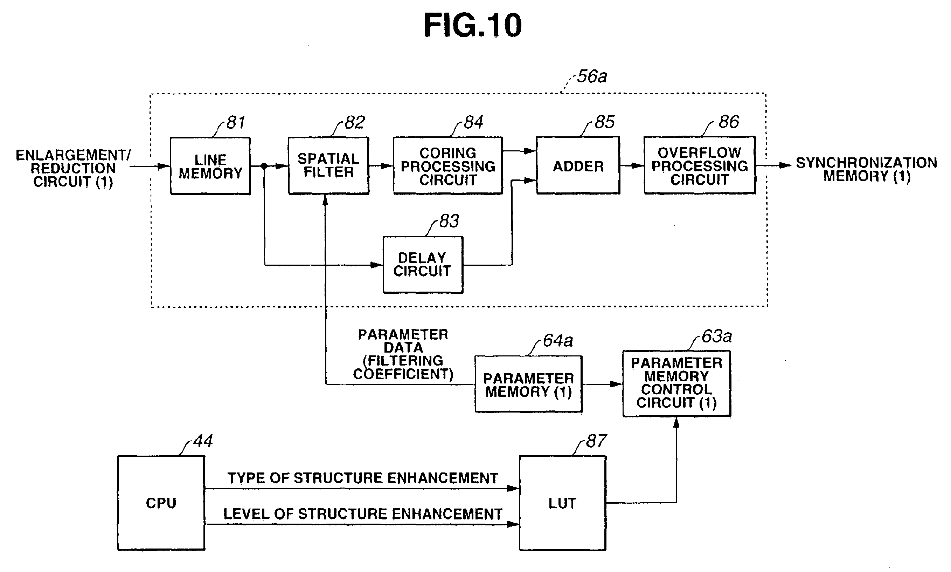

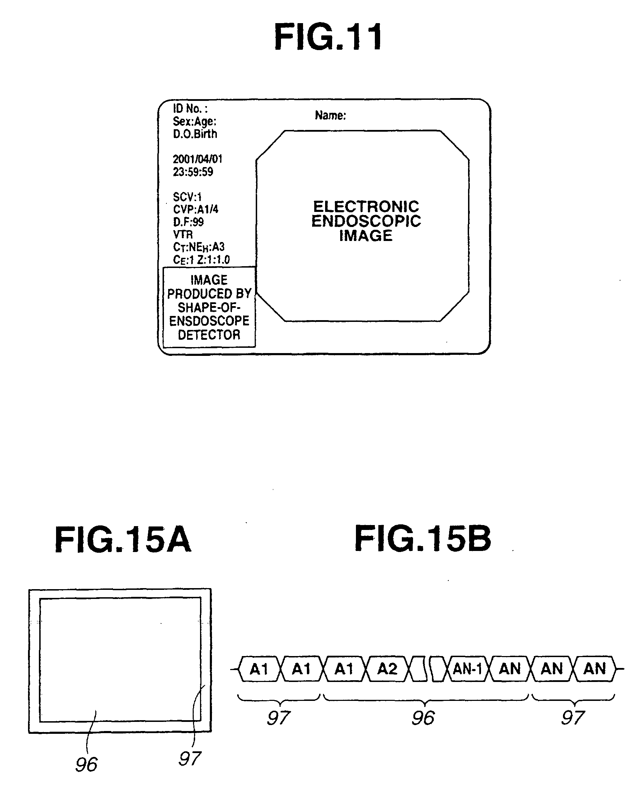

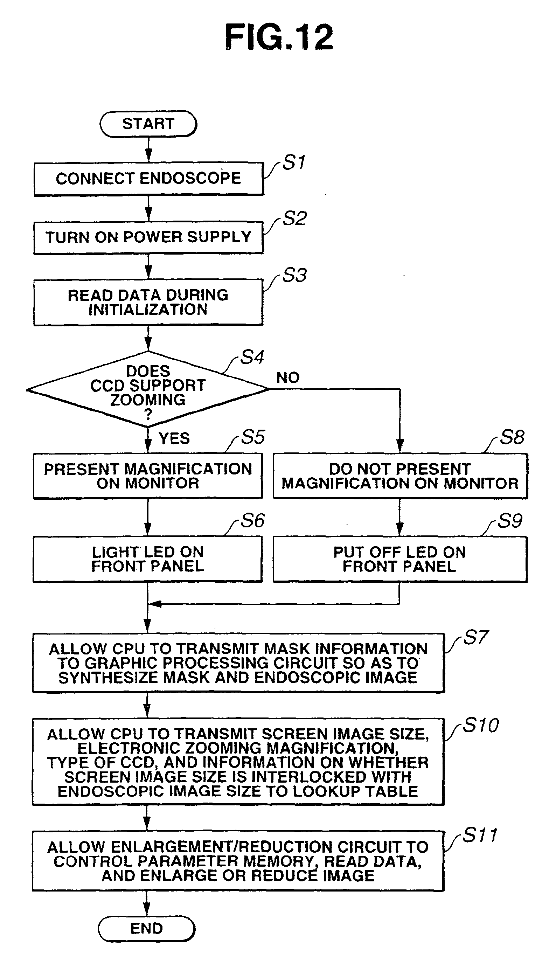

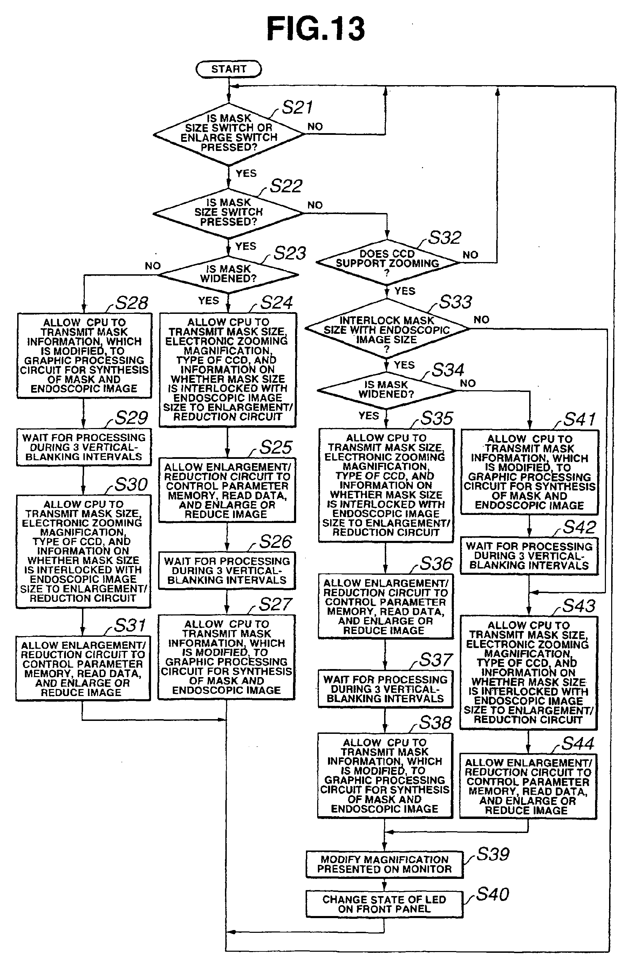

Fig. 1 to Fig. 13 are concerned with a first embodiment of the present invention. Fig. 1 shows the overall configuration of an electronic endoscope system in accordance with the first embodiment. Fig. 2 shows the circuit elements of an enlargement/reduction circuit. Fig. 3A and Fig. 3B are concrete examples of address data recorded in a lookup table. Fig. 4A, Fig. 4B, and Fig. 4C show concrete examples of a mask size (screen image size) in which an endoscopic image is displayed. Fig. 5A, Fig. 5B, and Fig. 5C show examples of an endoscopic image displayed in a semi-full mask size when enlargement is directed on the condition that an endoscopic image size should not be interlocked with a mask size. Fig. 6 shows an example of a menu screen image that helps a user determine various settings. Fig. 7 shows an example of the layout of components on a front panel. Fig. 8A, Fig. 8B, and Fig. 8C show examples of an endoscopic image displayed when enlargement is directed on the condition that an endoscopic image size should be interlocked with a mask size. Fig. 9 shows an example of a monitor screen image displayed when a CCD does not support electronic zooming. Fig. 10 shows the circuit elements of a structure enhancement circuit. Fig. 11 shows an example of display of an endoscopic image on which an image produced by a shape-of-endoscope detector is superimposed. Fig. 12 describes initialization. Fig. 13 describes major operations to be performed in the present embodiment when changing of mask sizes or enlargement is directed.

As shown in Fig. 1, an electronic endoscope system 1 in accordance with the first embodiment of the present invention comprises: an electronic endoscope 2 that is used for endoscopic examination; a light source unit 3 that supplies illumination light to the electronic endoscope (hereinafter, simply, an endoscope) 2; a video processor 4 serving as a signal processing unit that processes a signal produced by an imaging device incorporated in the endoscope 2; a monitor (1) 5A, a printer (1) 5B, a VTR (1) 5C, a photography system 5D, and a filing system 5E that are connected to the video processor 4 and operate on a standard video signal (SDTV in Fig. 1); a shape-of-endoscope detector 6 that detects the shape of an endoscope and transmits a video signal associated with the shape of an endoscope; an optional board 7 serving as an expansion board that is optionally loaded in the video processor 4 in order to extend the features implemented on a main board that perform basic signal processing; a monitor (2) 8A, a printer (2) 8B, and a VTR (2) 8C that are connected to the video processor 4 when the optional board 7 is loaded in the video processor 4 and that operate on a high-definition video signal (HDTV in Fig. 1); and a keyboard 9 connected to the video processor 4 and used to enter data or a directive.

The endoscope 2 has an elongated insertion unit 11 that is inserted into a body cavity. An operating unit 12 which an operator holds to insert the insertion unit or the like is formed at the rear end of the insertion unit 11. A light guide 13 over which illumination light is propagated to the inside of the insertion unit 11 is passed through the endoscope 2. A light guide connector 14 fixed to the rear end of the light guide 13 extended from the operating unit 12 to outside is freely detachably coupled to the light source unit 3.

A lamp 15 that emits white light is incorporated in the light source unit 3. Illumination light emanating from the lamp 15 passes through red, green, and blue filters that are formed circumferentially as parts of a rotary filter 17 which is rotated by a motor 16. The red, green, and blue filters have the properties of transmitting light rays that fall within the regions of the visible spectrum associated with red, green, and blue respectively. This results in field-sequential illumination light. A diaphragm 19 whose degree of opening is controlled by a diaphragm control circuit 18 regulates the amount of light. The resultant light is converged on a condenser lens 21, and is incident on the rear end of the light guide 13.

A motor control circuit 22 controls the motor 16 so that the motor 16 will be rotated at a predetermined rotating speed. Moreover, the diaphragm control circuit 18 is connected to a connector 24, which is coupled to the video processor 4, via a D/A converter 23. The diaphragm control circuit 18 receives a light regulation signal from a light regulation circuit that will be described later.

Illumination light emanating from the light source unit 3 and falling on the rear end of the light guide 13 is propagated along the light guide 13, and emitted forward while spreading from the distal end of the light guide 13 locked in an illumination window formed in a distal section 25 of the insertion unit 11. Consequently, an intracavitary lesion or any other object is-illuminated.

An objective lens 26 locked in an observation window adjoining the illumination window forms an optical image of the illuminated object within the distal section 25. A solid-state imaging device, or more particularly, a charge-coupled device (CCD) 27 is located at the position of the image plane of the objective. The CCD 27 photoelectrically converts the formed optical image.

A signal line extending from the CCD 27 is freely detachably coupled to the video processor 4 via a signal connector 28.

A driving signal sent from a drive circuit 32 included in a floating circuit block 31 isolated from a secondary circuit block 37 included in the video processor 4 is applied to the CCD 27. An imaging signal resulting from photoelectric conversion is read from the CCD 27, and transferred to a preamplifier 33 included in the video processor 4 via the connector 28.

A correlated double sampling (CDS) circuit 34 performs correlated double sampling on a signal amplified by the preamplifier 33 so as to convert the signal into a baseband video signal composed of sampled signal components. Thereafter, the video signal is transferred to an A/D converter 35 and converted into a digital signal.

The digital signal is transferred to an optical black (OB) clamping circuit 38 included in the secondary circuit block 37 via an insulation circuit 36 formed with a photocoupler and others, and also transferred to a light regulation circuit 39 that produces a light regulation signal which directs light regulation.

The light regulation signal sent from the light regulation circuit 39 passes through the insulation circuit 36 and is then converted into an analog light regulation signal by the D/A converter 23 included in the light source unit 3. The diaphragm control circuit 18 adjusts the degree of opening of the diaphragm 19 according to the light regulation signal, and automatically regulates an amount of illumination light so that a video signal will represent an appropriate brightness level.

The OB clamping circuit 38 reads a signal level from an optical black area of the CCD 27 so as to establish a black level. A digital video signal whose black level is established by the OB clamping circuit 38 is transferred to a white balance (W/B in Fig. 1) correction circuit 40, whereby white balance is performed, that is, red, green, and blue signal levels are balanced in order to establish a white level.

An automatic gain control (AGC) circuit 41 performs automatic gain control on a signal that has undergone white balance. The resultant signal is transferred to a freeze memory 42 in which an image is frozen. A freeze control circuit 43 controls the freeze memory 42. Moreover, the freeze control circuit 43 controls the operations of the components of the video processor 4. A CPU 44 that extends control according to the result of detecting performed by a detecting means that detects the connection to the video processor 4 controls the freeze control circuit 43.

Moreover, the operating unit 12 of the endoscope 2 has endoscope switches 45 including a Freeze switch. When the Freeze switch is handled, the directive signal is transferred to the CPU 44 via the insulation circuit 36. In response to the directive signal, the CPU 44 freezes image data read from the freeze memory 42 using the freeze control circuit 43.

Normally, a signal (image data) to be transferred to the freeze memory 42 is time-sequentially updated. However, when the Freeze switch is handled, the CPU 44 inhibits writing of image data in the freeze memory 42 using the freeze control circuit 43. Consequently, image data read from the freeze memory 42 remains unchanged, but frozen image data is displayed.

Freeze can be directed using the endoscope switch 45 included in the endoscope 2. Otherwise, a switch included in the keyboard 9 or a Freeze switch whose role is filled by a foot switch that is not shown may be handled. Even in this case, the freeze control circuit controls freeze under the control of the CPU 44.

Moreover, after the Freeze switch is used to freeze an image, if the Freeze switch is pressed again, the CPU 44 extends control so as to unfreeze an image.

Incidentally, the endoscope 2 includes an endoscope identification (ID) circuit 46 that produces endoscope identification information including the type of CCD 27 incorporated in the endoscope 2. The CPU 44 reads the endoscope identification information by way of the signal connector 28 and insulation circuit 36. The CPU 44 extends control so that a signal will be processed according to the type of CCD 27 incorporated in the endoscope 2 connected to the video processor 4.

For example, when a CCD offering a small number of pixels is employed, enlargement based on electronic zooming is restricted in order to thus restrict the processing that especially degrades image quality. In other words, restrictions are imposed in order to disable unnecessary handling.

Moreover, an optional board detection circuit 47 that detects identification information concerning the optional board 7 so as to detect the feature implemented on the optional board 7 is mounted on the optional board 7. The CPU 44 identifies the feature implemented on the loaded optional board 7 on the basis of the detection information of the optional board detection circuit 47, and performs an associated control operation.

When the optional board 7 is not loaded, restrictions are imposed in order to disable designation of an extension feature, which is implemented by loading the optional board 7, through a menu screen image that helps a user designate various features.

When the optional board 7 is loaded, a signal read from the freeze memory 42 is transferred to an IHb color enhancement circuit 48 mounted on the optional board 7, and applied to a contact a of a selector 49.

In this case, when the optional board 7 is loaded, the CPU 44 controls the selector 49 so that a contact that selects the connection to the optional board 7, that is, a contact b will conduct. Moreover, when the optional board 7 is unloaded, the key entry to be achieved using a certain button or key of a front panel 50 or the keyboard 9 is invalidated, or an LED indicating a certain feature is turned off. Moreover, a menu item included in a menu to be displayed with the press of a Menu key included in the keyboard 9 is, for example, hatched in order to disable designation of the feature that is implemented on the optional board 7. The hatching informs a user of the fact that the feature is invalid.

Referring to Fig. 1, when the optional board 7 is loaded, an output signal read from the freeze memory 42 is transferred to a tone regulation circuit 51 via the IHb color enhancement circuit 48 and a motion picture color smear correction circuit 52, which are mounted on the optional board 7, by way of the contact b of the selector 49.

In contrast, when the optional board 7 is unloaded, a signal read from the freeze memory 42 is transferred to the tone regulation circuit 51 by way of the contact a of the selector 49. In this case, neither IHb color enhancement nor motion picture color smear correction is carried out.

The IHb color enhancement circuit 48 calculates an IHb value (=32×Log(R/G)) that correlates to an amount of hemoglobin, and enhances a color using the IHb value. A change in the IHb value corresponds to a change in an amount of blood.

Moreover, since the present embodiment adopts a field-sequential imaging method according to which an object is imaged under field-sequential illumination light, a color smear may occur during imaging of a region that makes a fierce motion. According to the present embodiment, the motion picture color smear correction circuit 52 corrects such a color smear in a motion picture attributable to the field-sequential imaging method.

A signal whose component representing a tone is regulated by the tone regulation circuit 51 is transferred to a gamma correction circuit 53, and subjected to gamma correction. The signal having undergone gamma correction is processed by a succeeding SDTV signal processing system.

The signal having undergone gamma correction is transferred to an enlargement/reduction circuit (1) 55a. Electronic enlargement or reduction is performed at an enlargement ratio associated with the size of an endoscopic image to be displayed on the monitor (1) 5A.

The signal processed by the enlargement/reduction circuit (1) 55a undergoes enhancement such as structure enhancement or contour enhancement by a structure enhancement circuit (1) 56a. Thereafter, the signal is transferred to a synchronization memory (1) 57a. A field-sequential digital video signal having red, green, and blue color signal components and being sent from the structure enhancement circuit (1) 56a is time-sequentially written in the synchronization memory (1) 57a. The red, green, and blue color signal components are read simultaneously, whereby synchronization is achieved.

A character/mask/image synthesizer (1) 58a appends characters or a mask to an image represented by a signal that has the components thereof synchronized, or switches the signal and a menu or test signal (color bar signal or the like). The character/mask/image synthesizer (1) 58a handles characters or a mask under the control of a graphic processing circuit (1) 59a controlled by the CPU 44.

An output signal of the character/mask/image synthesizer (1) 58a is converted into analog red, green, and blue signals by a D/A converter (1) 60a, and transferred to a synthesizer (1) 61a that synthesizes an image represented by the signals with an image produced by the shape-of-endoscope detector 6 or switches an image produced by the filing system 5E. Thus, gain control that is not shown is performed on the signals. The resultant signals are transmitted to the monitor (1) 5A, printer (1) 5B, VTR (1) 5C, photography system 5D, and filing system 5E, which are compatible with the SDTV, via a 75 Ω drive circuit (1) 62a.

Moreover, the operations and settings of the AGC circuit 41, gamma correction circuit 53, enlargement/reduction circuit (1) 55a, and structure enhancement circuit (1) 56a are determined based on the parameter values recorded in a parameter memory (1) 64a that is controlled by a parameter memory control circuit (1) 63a.

The white balance correction circuit 40, AGC circuit 41, tone correction circuit 51, gamma correction circuit 53, enlargement/reduction circuit (1) 55a, and structure enhancement circuit (1) 56a are controlled by the CPU 44.

Moreover, when the optional board 7 is loaded, a signal having undergone gamma correction is processed by a HDTV signal processing system implemented on the optional board 7.

Specifically, a structure enhancement circuit (2) 56b performs structure enhancement on a signal having undergone gamma correction. An enlargement/reduction circuit (2) 55b enlarges or reduces an image, which is represented by the resultant signal, at an electronic zooming magnification associated with the size of an endoscopic image to be displayed on the monitor (2) 8A.

A sequence followed by the HDTV signal processing system is different from that followed by the SDTV signal processing system. This is because an enlargement ratio at which the HDTV signal processing system enlarges an image is larger than the one at which the SDTV signal processing system does. Therefore, if structure enhancement is preceded by enlargement, a filter required should be large in size. Nevertheless, similarly to the sequence followed by the SDTV signal processing system, enlargement or reduction may precede structure enhancement.

A video signal representing an image enlarged or reduced at an electronic zooming magnification has its color signal components synchronized in a synchronization memory (2) 57b, and is then transferred to a character/mask/image synthesizer (2) 58b. Characters or a mask is then appended to the image, or the video signal is switched to a menu or test signal (color bar signal).

The character/mask/image synthesizer (2) 58b handles characters or a mask under the control of a graphic processing circuit (2) 59b that is controlled by the CPU 44.

An output signal of the character/mask/image synthesizer (2) 58b is digital-to-analog converted by a D/A converter (2) 60b, whereby HDTV analog red, green, and blue signals are produced. The red, green, and blue signals are transferred to a synthesizer (2) 61b that synthesizes an image represented by the signals with an image produced by the shape-of-endoscope detector 6 or switches the image and an image produced by a filing system (2) 8E. Gain control and others not shown are also performed on the signals. Thereafter, the resultant signals are transmitted to the monitor (2) 8A, the printer (2) 8B, and the VTR (2) 8C, which are compatible with the HDTV, by way of a 75 Ω drive circuit 62b.

Moreover, the structure enhancement circuit (2) 56b and the enlargement/reduction circuit (2) 55b have the settings thereof for structure enhancement or an electronic zooming magnification for enlargement or reduction determined based on the parameter values stored in a parameter memory (2) 64b that is controlled by a parameter memory control circuit (2) 63b.

The synthesizer (1) 61a transmits an output signal of the filing system 5E to the SDTV monitor (1) 5A after switching a signal sent from the endoscope 2 into the output signal. Likewise, the synthesizer (2) 61b switches the signal sent from the endoscope 2 into the output signal of the filing system 5E and transmits the output signal to the (HDTV) monitor (2) 8A.

The (HDTV) monitor (2) 8A employed in the present embodiment can cope with both the SDTV and HDTV video signals. Therefore, normally, the monitor (2) 8A is used mainly to handle the HDTV video signal. If necessary, the monitor (2) 8A deals with an output signal of the filing system 5E so as to check the output signal of the filing system 5E.

Fig. 2 shows the circuit elements of the enlargement/reduction circuit (1) 55a and the peripherals.

The enlargement/reduction circuit (1) 55a comprises selectors 71, 72, and 73, a frame memory 74, a transformation circuit 75 that performs conversion (more particularly, interpolation), and a signal generator (SSG) 76.

The signal generator 76 is responsible for switching of the contacts a and b of each of the selectors 71 to 73, reading of data from the frame memory 74, and control of the transformation circuit 75.

An output signal of the gamma correction circuit 51 is applied to the contact a of the selector 71 and the contact b of the selector 72. An output signal of the transformation circuit 75 is applied to the contact b of the selector 71. An output of the selector 71 is stored in the frame memory 74. A signal read from the frame memory 74 is applied to both the contact b of the selector 73 and the contact a of the selector 72. Moreover, an output of the selector 72 has the signal components thereof interpolated or thinned by the transformation circuit 75. An output signal of the transformation circuit 75 is applied to the contact a of the selector 73.

For enlargement, the signal generator 76 selects the contacts a of the selectors 71 to 73 respectively. For reduction, the contacts b thereof are selected. When enlargement is designated as shown in Fig. 2, image data sent from the gamma correction circuit 51 and representing one screen image is temporarily stored in the frame memory 74. The transformation circuit 75 intermittently reads the image data from the frame memory 74 and linearly interpolates the data items so as to thus achieve enlargement. The enlarged image data is transmitted from the selector 73 to the structure enhancement circuit (1) 56a.

For reduction, image data sent from the gamma correction circuit 51 has the data items thereof linearly interpolated by the transformation circuit 75, and is intermittently (thinned and) written in the frame memory 74. The image data read from the frame memory 74 is transmitted from the selector 73 to the structure enhancement circuit (1) 56a.

A mask size (screen image size), an electronic zooming magnification, a type of CCD, and information on whether the mask size is interlocked with an endoscopic image size or screen image size (so that the mask size will be changed based on a zooming magnification) are transferred from the CPU 44 to a lookup table (LUT) 77. Data read from the lookup table 77 is transmitted to the parameter memory (1) 64a via the parameter memory control circuit (1) 63a.

A control signal based on which the frame memory 74 is controlled and an interpolation coefficient used for enlargement or reduction are recorded in the parameter memory (1) 64a. Parameter data read from the parameter memory (1) 64a is transferred to the signal generator 76. The signal generator 76 then controls the frame memory 74, transformation circuit 75, and selectors 71 to 73.

Fig. 3A shows a concrete example of address data that is written in the lookup table 77 and that represents an address in the parameter memory (1) 64a from which data should be read. Fig. 3B shows a concrete example of address data representing an address in the parameter memory (1) 64b from which data should be read in case the data is the HDTV signal. The parameter data that is used for the SDTV and that is shown in Fig. 3A is different from the parameter data that is used for the HDTV and shown in Fig. 3B. Incidentally, the circuit elements and operations of the enlargement/reduction circuit (2) 55b are nearly identical to those of the enlargement/reduction circuit (1) 55a.

Moreover, the present embodiment makes, for example, three mask sizes (screen image sizes), in which an endoscopic image is displayed, available, and allows a user to select any of the mask sizes.

Fig. 4A, Fig. 4B, and Fig. 4C show the monitor screen image areas having the medium, semi-full, and full-height mask sizes listed in Fig. 3A and Fig. 3B. For example, the monitor screen image size for the SDTV monitor (1) 5A is selected from among the medium, semi-full, and full-height mask sizes that get larger in that order as shown in Fig. 4A to Fig. 4C. The full-height size provides an octagonal mask area in which an endoscopic image is displayed fully using the height of the display screen of the monitor (1) 5A. A user can select any of the mask sizes or screen image sizes he/she likes.

In this case, a region imaged by the CCD 27 is the same despite the difference in a mask size. An endoscopic image is displayed in a selected mask size using almost all the effective pixel locations of the CCD 27.

Moreover, according to the present embodiment, the display form in which character information such as patient data is displayed is varied depending on which of the mask sizes is selected. For example, as shown in Fig. 4A, when the medium mask size is selected, a relatively large area in which character information such as patient data is displayed can be preserved by the left side of the mask area or screen image area. when the semi-full mask size shown in Fig. 4B is selected, the area in which patient data or the like is displayed gets narrowed. When the full-height mask size shown in Fig. 4C is selected, the area gets further narrowed.

Consequently, according to a selected mask size, the CPU 44 changes the display modes in which character information such as patient data is displayed as shown in Fig. 4A to Fig. 4C. Especially, when the semi-full or full-height size is selected, character information such as patient data is prevented from being displayed within the mask area or screen image area. Thus, the character information is hindered from being superimposed on an endoscopic image displayed within the mask area.

Moreover, when the semi-full size is selected, character information may be arranged so that it will not be superimposed on an endoscopic image.

Referring to Fig. 4A, Fig. 4B, and Fig. 4C, character information is displayed in the same display mode between the semi-full and full-height sizes. In the full-height size, the character information display area may be further compressed.

The mask or screen image sizes for displaying an HDTV image are slightly different from those for displaying an SDTV image but nearly identical thereto.

Referring back to Fig. 3A and Fig. 3B, electronic zooming is enabled or disabled depending on the type of CCD 27 (four types of CCDs are listed) as listed in Fig. 3A and Fig. 3B.

For example, when the CCD 27 is of type 1, if the SDTV video signal is produced, the mask sizes are limited to the medium and full-height sizes alone and the electronic zooming magnification Z is limited to 1.0 alone.

When the CCD 27 is of type 1, the number of pixels offered by the CCD 27 is smaller than those offered by the other types of CCDs. If the electronic zooming magnification is raised, degradation in image quality becomes conspicuous. Therefore, enlargement based on electronic zooming is restricted. Moreover, when the CCD 27 is of type 1, the selectable mask sizes are limited in order to prevent signal processing from getting complex.

On the other hand, even when the CCD 27 is of type 1, if the HDTV video signal is produced, any of three mask sizes of the medium, semi-full, and full-height sizes can be selected. Even in this case, the electronic zooming magnification Z is limited to 1.0 alone, though.

Moreover, when the CCD 27 is of type 3 that offers a larger number of pixels than the type 1, if the SDTV video signal is produced, the electronic zooming magnification Z can be set to any of 1.0, 1.4, 1.6, 1.8, and 2.2. Whether a mask size is interlocked with an endoscopic image size will be described later with reference to Fig. 8A, Fig. 8B, and Fig. 8C.

Fig. 5A, Fig. 5B, and Fig. 5C show examples of display in a monitor screen image area having, for example, the semi-full mask size on the condition that an endoscopic image size is not interlocked with a mask size. Specifically, Fig. 5A to Fig. 5C show endoscopic images displayed with the electronic zooming magnification Z set to 1.0, 1.6, and 2.2 respectively.

When the electronic zooming magnification Z is changed from 1.0 to 1.6 or 2.2, that is, from Fig. 5A to Fig. 5B or Fig. 5C, the center portion of Fig. 5A, that is, an endoscopic image is enlarged with the semi-full mask size held intact. Specifically, when the endoscopic image in the center of the screen image area shown in Fig. 5A is enlarged or electrically zoomed at a magnification of 1.6 times larger, the resultant endoscopic image having an aspect ratio of 1:1.6 is displayed as shown in Fig. 5B. When the endoscopic image in center of the screen image area shown in Fig. 5A is enlarged or electrically zoomed at a magnification of 2.2, the resultant endoscopic image whose length and width correspond to the halves of the mask size is displayed as shown in Fig. 5C.

Actual selection of a value from the numerous values of the electronic zooming magnification Z listed in Fig. 3A and Fig. 3B, or changing of the mask sizes is achieved through a menu screen image shown in Fig. 6.

A plurality of settings can be determined by specifying information in a plurality of menu items contained in the menu screen image shown in Fig. 6.

For example, features to be assigned to the plurality of endoscope switches 45 included in the endoscope 2 can be selected or designated. Moreover, a structure enhancement level and an IHb color enhancement level can be designated. A photometry mode in which light regulation is performed, an area whose data is used to calculate an IHb value, and electrical zooming magnifications to be associated with a plurality of modes can be designated. Moreover, whether a mask size is interlocked with an endoscopic image size to be changed through electronic zooming (On or Off) can be selected. Furthermore, changing of the mask sizes can be designated.

Incidentally, Fig. 6 shows an example of settings determined with the optional board 7 loaded. When the optional board 7 is unloaded, a menu item concerning an extension feature to be implemented on the optional board 7, for example, a menu item concerning calculation of an IHb value is, for example, hatched in order to prevent specification of any value in the item. Owing to the hatching, a user readily gets aware of the fact that the feature is invalid.

The total number of combinations of modes where mask sizes can be changed including combinations of unchanged mask sizes is nine as follows:
medium ←→ medium
medium ←→ semi-full
semi-full ←→ medium
medium ←→ full-height
full-height ←→ medium
semi-full ←→ semi-full
semi-full ←→ full-height
full-height ←→ semi-full
full-height ←→ full-height
The mask sizes listed on the left side above are the mask sizes or screen image sizes designated when the power supply is turned on.

When a Screen Image Size key on the keyboard 9 or an endoscope switch 45 is pressed, the mask sizes can be changed. This setting can be determined independently for the SDTV and HDTV.

Consequently, for example, when observation is performed according to the HDTV and recording is performed according to the SDTV, the screen image sizes for an HDTV image being viewed may be changed but the screen image sizes for an SDTV image to be recorded may be left unchanged.

Moreover, a menu item for enlargement shown in Fig. 6 includes Level 0, Level 1 and Level 2 fields in which electronic zooming levels 1 and 2 are specified.
Enlargement ratios are assigned with level 1 and level 2. (Incidentally, electronic zooming level 0 is fixed to a magnification of 1.0.)

The enlargement ratios are selected or designated through the menu screen image. The enlargement ratios to be assigned to level 1 and level 2 are selected from among 1.4, 1.6, 1.8, and 2.2 listed in Fig. 3A and Fig. 3B. Referring to Fig. 6, 1.0, 1.6, and 2.2 are selected or designated. In this case, when enlargement is directed, the images are displayed as shown in Fig. 5A, Fig. 5B, and Fig. 5C respectively.

In order to direct enlargement based on electronic zooming, an Enlarge switch 50a included in the front panel 50 shown in Fig. 7 or the endoscope switch 45 is pressed.

Three LEDs are arranged above the Enlarge switch 50a on the front panel 50 shown in Fig. 7. When the Enlarge switch 50a is pressed, one of the LEDs 50 associated with levels 0, 1, and 2 to which the enlargement ratios are assigned and located above the Enlarge switch 50a is lit.

Moreover, as shown in Fig. 5A to Fig. 5C, an enlargement ratio is presented on the monitor screen. The enlargement ratio is a magnification relative to 1.0 that is a reference.

Since the electronic zooming magnification is presented on the monitor screen, the fact that enlargement has been performed is clearly informed. When enlargement is needed, electronic zooming is performed. Normally, electronic zooming is therefore unemployed. The fact that electronic zooming is under way can be clearly informed, whereby a user is prevented from forgetting to invalidate the electronic zooming feature. The user will therefore not continue observation with an image kept zoomed.

Next, interlocking a mask size with an endoscopic image size will be described below.

When electronic zooming is designated, even if the full-height mask size is not selected, the full-height mask size is adopted. For this setting, On is specified in a Changing Sizes field in the menu screen image shown in Fig. 6.

As mentioned above, even if the mask sizes are changed, a region imaged by the CCD 27 is the same.

When the electronic zooming magnification Z is set to 1.0, almost all the effective pixel locations of the CCD 27 are employed. Therefore, the mask size cannot be increased any more. However, when enlargement based on electronic zooming is under way, part of an image formed by the CCD 27 is displayed. Therefore, the mask size can be increased, and part of an image formed in a used area of the CCD 27 can be enlarged and displayed.

Fig. 8A, Fig. 8B, and Fig. 8C show examples of display in a monitor screen image area on the condition that enlargement is performed with a mask size interlocked with an endoscopic image size. For example, the examples of display are attained when the electronic zooming magnification Z is changed to 1.6 and 2.2 with the semi-full size selected.

As shown in Fig. 8A, when the electronic zooming magnification Z is changed from 1.0 to 1.6 or 2.2, an endoscopic image is, as shown in Fig. 8B and Fig. 8c, enlarged and displayed in the full-height mask size.

When a mask size is increased while being interlocked with an endoscopic image size, part of an endoscopic image that cannot be displayed in the semi-full size can be displayed. This is helpful in observation. Even when it is designated that a mask size is interlocked with an endoscopic image size, the full-height size remains unchanged.

Specifically, when a mask size is interlocked with an endoscopic image size to be changed due to enlargement based on electronic zooming, a view image area in which an electronically zoomed image is displayed can be widened.
The view image area is therefore made wider than when a view range is not interlocked with en endoscopic image size to be changed due to electronic zooming.

As described in conjunction with Fig. 3A and Fig. 3B, when the CCD is of type 1, electronic zooming is restricted. Moreover, the CCD of type 1 does not support the semi-full mask size.

An LED located above the Enlarge switch 50a on the front panel 50 is left unlit in order to inform that electronic zooming is disabled. Moreover, an enlargement ratio is not presented on the monitor screen. Fig. 9 shows the monitor screen. As shown in Fig. 9, Z denoting the electronic zooming magnification is not displayed.

The CCD 27 of type 2 does not support electronic zooming. Therefore, the LED located on the front panel 50 is left unlit in order to inform that electronic zooming is disabled. Moreover, an enlargement ratio is not presented on the monitor screen.

As mentioned above, according to the present embodiment, enlargement based on electronic zooming is restricted according to the type of CCD 27 incorporated in the endoscope 2 actually connected to the video processor 4.
The indication given by a member used to direct enlargement is restricted, and whether the feature of enlargement is valid is clearly informed in order to prevent a user from directing enlargement by mistake. This contributes to improvement of maneuverability.

Fig. 10 shows the circuit elements of the structure enhancement circuit (1) 56a.

A signal sent from the enlargement/reduction circuit (1) 55a is transferred to each of a spatial filter 82 formed with a matrix circuit and a delay circuit 83 that delays a signal via a line memory 81. The line memory 81, spatial filter 82, and delay circuit 32 are included in the structure enhancement circuit (1) 56a. The spatial filter 82 passes signal components, of which spatial frequencies are higher than a certain spatial frequency, (samples a signal component representing an edge) according to parameter data (more particularly, a filtering coefficient used for matrixing) read from the parameter memory (1) 64a.

An output signal of the spatial filter 82 has feeble signal components thereof reduced by a coring processing circuit 84, and is then transferred to an adder 85. The resultant signal is added to a signal delayed by the delay circuit 83. Furthermore, an overflow processing circuit 86 deals with an overflow of the output of the adder 85 so as to produce a signal that has undergone structure enhancement, and transmits the signal to the synchronization memory (1) 57a in the next stage.

Data representing the type and level of structure enhancement is transferred from the CPU 44 to a lookup table 87 included for structure enhancement. Address data representing an address of parameter data is read from the lookup table 87, and transmitted to the parameter memory control. circuit (1) 63a. The parameter memory control circuit (1) 63a reads parameter data (filtering coefficient) from the address in the parameter memory (1) 64a represented by the address data, and transmits the parameter data to the spatial filter 82.

As mentioned above, structure enhancement is performed based on the type or level designated through the menu screen image.

Moreover, according to the present embodiment, a shape-of-endoscope detection image produced by the shape-of-endoscope detector 6 can be, as shown in Fig. 11, displayed on the monitor screen while being superimposed on an endoscopic image. Specifically, a video signal representing a shape-of-endoscope detection image that depicts a model of the shape of the insertion unit 11 of the endoscope 2 detected by the shape-of-endoscope detector 6 is transferred from the shape-of-endoscope detector 6 to the synthesizer (1) 61a or synthesizer (2) 61b shown in Fig. 1. A superimposition circuit included in the synthesizer superimposes the video signal on the video signal that represents an endoscopic image and that is sent from the D/A converter (1) 60a or D/A converter (2) 60b. Consequently, the shape-of-endoscope detection image is displayed in a picture-in-picture (PinP) form as shown in Fig. 11.

According to the present embodiment, the characteristic of a filter concerning contour enhancement to be performed on an SDTV signal and the characteristic of a filter concerning contour enhancement to be performed on an HDTV signal are optimized independently of each other and therefore different from each other.

Moreover, according to the present embodiment, the aspect ratio for the HDTV can be selected from 4:3 and 16:9.

Furthermore, the enhancement levels for IHb color enhancement or structure enhancement can be changed using a switch on the front panel 50. Levels to be associated with switches with switches 1, 2, and 3 on the front panel 50 can be designated through the menu screen image shown in Fig. 6.

Moreover, the levels associated with the switches may be interlocked with each other and associated to one switch.

Specifically, an Enhance Color switch can be used. When the switch is pressed, the structure enhancement levels and color enhancement levels associated with the mode are switched at the same time. At this time, an LED of an Enhance Structure switch is put off.

Next, initialization to be performed when the electronic endoscope system in accordance with the present embodiment is put into operation will be described with reference to Fig. 12.

After the endoscope 2 is connected to the video processor 4 at step S1, if the power supply of the video processor 4 is turned on at step S2, the CPU 44 included in the video processor 4 performs reading for initialization at step S3.

At step S4, it is determined whether the CCD 27 incorporated in the endoscope 2 actually connected to the video processor 4 supports zooming. The determination is performed based on identification information sent from the endoscope ID circuit 46.

When it is determined that the CCD supports zooming, an enlargement ratio for electronic zooming is presented on the monitor (1) 5A at step S5. Moreover, an associated LED on the front panel 50 is lit at step S6, and control is passed to step S7.

On the other hand, when it is determined that the CCD does not support zooming, an enlargement ratio for electronic zooming is not presented on the monitor (1) 5A at step S8. Moreover, the associated LED on the front panel 50 is put off at step S9, and control is returned to step S7.

At step S7, the CPU 44 transmits mask information to the graphic processing circuit (1) 59a. The character/mask/image synthesizer (1) 58a synthesizes a mask and an endoscopic image.

At step S10, the CPU 44 transmits a screen image size, an electronic zooming magnification, a type of CCD, and information on whether the screen image size is interlocked with an endoscopic image size to the lookup table 77 included in the enlargement/reduction circuit (1) 55a.

The enlargement/reduction circuit (1) 55a controls the parameter memory (1) 64a on the basis of an output of the lookup table 77, reads parameter data, and enlarges or reduces an image. Thus, the initialization is completed.

Next, referring to Fig. 13, operations to be performed when a Mask Size (screen image size) switch or an Enlarge switch is pressed will be described below.

After initialization is performed as described in Fig. 12, the CPU 44 determines a step S21 described in Fig. 13 whether the Mask Size switch and Enlarge switch 50a are pressed. When the switches are not pressed, the CPU 44 waits until they are pressed. When the Enlarge switch 50a is pressed, the CPU 44 determines at step S22 whether the Mask Size switch is pressed. When the Mask Size switch is pressed, the CPU 44 determines at step S23 whether it is directed to widen a mask (to increase a mask size).

When it is directed to increase a mask size, the CPU 44 transmits a mask size, an electronic zooming magnification, a type of CCD, and information on whether the mask size is interlocked with an endoscopic image size to the enlargement/reduction circuit (1) 55a.

At step S25, the enlargement/reduction circuit (1) 55a controls the parameter memory (1) 64a, reads data therefrom, and then enlarges or reduces an image (enlarges an image in this case). At step S26, completion of processing is waited during three vertical-blanking intervals (3V in Fig. 13).

Thereafter, at step S27, the CPU 44 transmits mask information, which will be modified, to the graphic processing circuit (1) 59a, and synthesizes a mask and an endoscopic image. Thereafter, control is returned to step S21.

According to the present embodiment, since field-sequential illumination and imaging are adopted, when enlargement or reduction is performed based on electronic zooming, three vertical-blanking intervals are required to produce one synchronized color image. However, enlargement or reduction itself can be completed during one vertical-blanking interval, and mask sizes can be changed during a time much shorter than one vertical-blanking interval.

Consequently, when enlargement or reduction and changing of mask sizes are performed simultaneously, even when the mask sizes are changed, an unexpected image may be displayed instantaneously, though, along with the changing of mask sizes until a color image to be enlarged or reduced is produced. Therefore, a wait state persists during three vertical-blanking intervals for the purpose of preventing display of an unnecessary screen image.

Specifically, mask information is transmitted to the graphic processing circuit (1) 59a at step S27. When a mask size is increased, enlargement is started before the start of three vertical-blanking intervals. Therefore, enlargement of one color image is already completed. Eventually, an enlarged color image is displayed in a mask area or screen image area having an increased mask size or screen image size without instantaneous display of an unnecessary image. It will be described later that a wait state persists during three vertical-blanking intervals.
The reason for this is the same as the aforesaid one.

On the other hand, when it is not directed at step S23 to increase a mask size, that is, when it is directed to decrease a mask size, mask information to be modified is transmitted to the graphic processing circuit (1) 59a at step S28. A mask and an endoscopic image are then synthesized. Thereafter, completion of processing is waited during three vertical-blanking intervals (3V in Fig. 13) at step S29. Consequently, one color image is produced through synchronization of three color signal components.

Thereafter, at step S30, the CPU 44 transmits a screen image size, an electronic zooming magnification, a type of CCD, and information on whether the screen image size is interlocked with an endoscopic image size to the enlargement/reduction circuit (1) 55a.

At step S31, the enlargement/reduction circuit (1) 55a controls the parameter memory (1) 64a, reads data, and enlarges or reduces an image (reduces an image in this case). Thereafter, control is returned to step S21.

When the Mask Size switch is not pressed at step S22, that is, when the Enlarge switch is pressed, control is passed to step S32. It is determined whether the CCD supports zooming.

When the CCD does not support zooming, control is returned to step S21. When the CCD supports zooming, control is passed to step S33. It is determined whether a mask size or screen image size is interlocked with an endoscopic image size. When a mask size is interlocked with an endoscopic image size, it is determined at step S34 whether the mask size is increased.

When the mask size is increased, the CPU 44 transmits a screen image size, an electronic zooming magnification, a type of CCD, and information on whether the screen image size is interlocked with an endoscopic image size to the enlargement/reduction circuit (1) 55a.

At step S36, the enlargement/reduction circuit (1) 55a controls the parameter memory (1) 64a, reads data, and enlarges or reduces (enlarges in this case) an image. Thereafter, at step S37, a wait state persists during three vertical-blanking intervals.

Thereafter, at step S38, the CPU 44 transmits mask information, which will be modified, to the graphic processing circuit (1) 59a, and synthesizes a mask and an endoscopic image. Thereafter, at step S39, the CPU 44 modifies the presentation of the magnification on the monitor (1) 5A.

Moreover, the LEDs on the front panel 50 to be lit are changed at step S40, and control is returned to step S21.

When it is not directed at step 34 to increase a mask size, but it is directed thereat to decrease a mask size, the CPU 44 transmits mask information, which will be modified, to the graphic processing circuit (1) 59a at step S41. The CPU 44 then synthesizes a mask and an endoscopic image. Thereafter, at step S42, the CPU 44 waits for completion of processing during three vertical-blanking intervals (3V in Fig. 13).

Thereafter, at step S43, the CPU 44 transmits a screen image size, an electronic zooming magnification, a type of CCD, and information on whether the screen image size is interlocked with an endoscopic image size to the enlargement/reduction circuit (1) 55a.

At step S44, the enlargement/reduction circuit (1) 55a controls the parameter memory (1) 64a, reads data, and enlarges or reduces an image. Thereafter, control is returned to step S39.

When it is found at step S33 that a screen image size is not interlocked with an endoscopic image size, that is, Off is specified, mask sizes or screen image sizes are not changed but control is passed to step S43.

According to the present embodiment, the endoscope 2 actually connected to the video processor 4 serving as a signal processing unit or the optional board 7 is detected. Based on the result of detecting, the CPU 44 extends control to restrict signal processing to be performed by the video processor 4 or restricts the indication that a feature is under way. Thus, maneuverability is improved for user's ease of use. This results in an easy-to-use environment.

For example, when the endoscope 2 in which the CCD 27 offering a small number of pixels is incorporated is connected, an electronic zooming feature is restricted in order to disable enlargement based on electronic zooming. Moreover, the indication that the feature is invalid is presented in order to inform a user of the fact that the feature is invalid.

Thus, the user is prevented from activating the feature by mistake. Moreover, assuming that an image is enlarged based on electronic zooming in an electronic endoscope system in accordance with a related art, if degradation in image quality is conspicuous, enlargement is canceled. This kind of time-consuming operation can be avoided.

### (Second Embodiment)

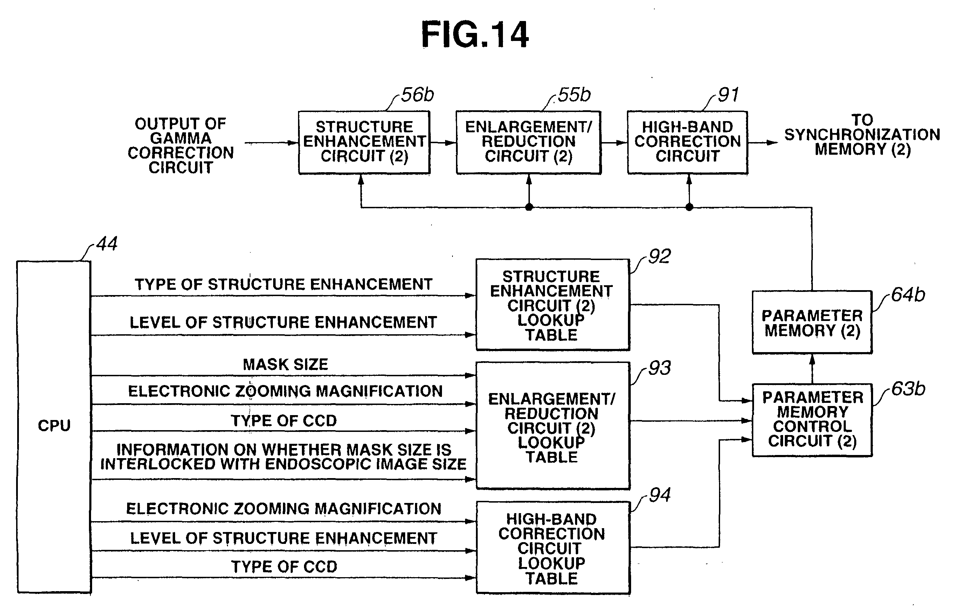

Next, a second embodiment of the present invention will be described with reference to Fig. 14, Fig. 15A, and Fig. 15B. Fig. 14 shows the circuit elements mounted on an optional board on which an extension feature is implemented according to the second embodiment.

According to the first embodiment, a signal processed by the structure enhancement circuit (2) 56b and enlargement/reduction circuit (2) 55b is transferred to the synchronization memory (2) 57b. According to the present embodiment, a high-band correction circuit 91 is mounted on the optional board 7 so that a signal processed by the enlargement/reduction circuit (2) 55b can be transferred to the synchronization memory (2) 57b via the high-band correction circuit 91 that corrects a high frequency band.

Moreover, the CPU 44 transfers a type of structure enhancement and a level thereof to a structure enhancement circuit (2) lookup table 92. The CPU 44 reads from the structure enhancement circuit (2) lookup table 92 address data that indicates an address of parameters based on which structure enhancement is performed by the structure enhancement circuit (2) 56b, and transfers the address data to the parameter memory control circuit (2) 63b. The parameter memory control circuit (2) 63b reads data, on which structure enhancement is performed, from the parameter memory (2) 64b, and transmits the data to the structure enhancement circuit (2) 56b for structure enhancement.

Moreover, the CPU 44 transfers a mask size, an electronic zooming magnification, a type of CCD, and information on whether a mask size is interlocked with an endoscopic image size to an enlargement/reduction circuit (2) lookup table 93. The CPU 44 reads address data, which indicates an address of parameters based on which the enlargement/reduction circuit (2) 55b enlarges or reduces an image, from the enlargement/reduction circuit (2) lookup table 93, and transfers the address data to the parameter memory control circuit (2) 63b. The parameter memory control circuit (2) 63b reads data, on which enlargement or reduction is performed, from the parameter memory (2) 64b, and transmits the data to the enlargement/reduction circuit (2) 55b for enlargement or reduction.

Moreover, the CPU 44 transfers an electronic zooming magnification, a structure enhancement level, and a type of CCD to a high-band correction circuit lookup table 94. The CPU 44 reads address data, which indicates an address of parameters based on which the high-band correction circuit 91 corrects a high frequency band, from the high-band correction circuit lookup table 94, and transfers the address data to the parameter memory control circuit (2) 63b. The parameter memory control circuit (2) 63b reads data, of which high frequency band data is corrected, and transmits the data to the high-band correction circuit 91 for high-band correction.

Owing to the foregoing circuit elements, a high-frequency band component of a signal sent from the enlargement/reduction circuit (2) 55b, of which level is lowered, can be corrected.

Moreover, according to the present embodiment, when structure enhancement is performed, dummy pixels 97 are appended to the margin of an image 96 whose structure is about to be enhanced. Thereafter, the structure enhancement is performed.

In this case, image data on which structure enhancement is performed is read from the memory. At this time, border pixels (herein A1 and AN) are repeatedly sampled from among pixels Al to AN constituting the image 96 as shown in Fig. 15B. The dummy pixels 97 are, as shown in Fig. 15B, appended to the border pixels. The resultant image data is transferred to the structure enhancement circuit (1) 56a or structure enhancement circuit (2) 56b, whereby structure enhancement is achieved.

According to the first embodiment, the spatial filter included in the structure enhancement circuit (1) 56a or structure enhancement circuit (2) 56b enhances a signal component representing an edge. In this case, a border between a frame and a blanking is enhanced unnaturally.

Therefore, according to the present embodiment, the dummy pixels 97 are appended for the purpose of structure enhancement.

Consequently, what is unnaturally enhanced is the border of the dummy pixels 97. Therefore, an actual image displayed within a mask area will not be affected at all. A naturally enhanced image is displayed.

As described in relation to the first embodiment, when the HDTV monitor (2) 8A is compatible with both the SDTV and HDTV, the video processor 4 changes the operating modes of the monitor so that an SDTV signal representing a digital file produced by the filing system (1) 5E can be visualized. The synthesizer (2) 61b may be designed to deal with not only the digital file produced by the filing system (1) 5E but also a video output of other VTR or printer.

The present embodiment provides nearly the same advantages as the first embodiment. In addition, when an image to be displayed on a display means is enhanced, unnatural enhancement of the border of an image is prevented.

### Industrial Applicability

As described so far, en electronic endoscope system in accordance with the present invention is useful in zooming and displaying an image produced by an electronic endoscope. The electronic endoscope system is suitable for display of an endoscopic image in, for example, a plurality of different screen image sizes.

## Claims

1. An electronic endoscope system comprising:
a signal processing unit that converts a signal read from a solid-state imaging device incorporated in an electronic endoscope into a predetermined video signal;
a detector that detects the connection to the signal processing unit; and
a restricting unit that restricts processing to be performed by the signal processing unit according to the result of detecting performed by the detector.

2. An electronic endoscope system according to Claim 1, wherein the detector detects the type of solid-state imaging device incorporated in the electronic endoscope connected to the signal processing unit.

3. An electronic endoscope system according to Claim 1, wherein: the signal processing unit includes a main board on which basic features are implemented, and an expansion board which is attachable or detachable to or from the main board and on which extension processing is performed on an image signal processed by the basic features; and the detector detects whether the expansion board is present.

4. An electronic endoscope system according to Claim 1, wherein the restricting unit that restricts processing restricts a selective item to be contained in a setting screen image that helps a user determine settings for the processing to be performed by the signal processing unit.

5. An electronic endoscope system according to Claim 1, wherein the restricting unit that restricts processing restricts handling of a switch on the front panel of the signal processing unit or lighting of an LED on the front panel.

6. An electronic endoscope system according to Claim 1, wherein the restricting unit that restricts processing restricts handling of a switch on the keyboard of the signal processing unit or lighting of an LED on the front panel.

7. An electronic endoscope system according to Claim 1, wherein the processing to be performed by the signal processing unit is electronic zooming.

8. An electronic endoscope system according to Claim 1, wherein the restricting unit restricts indication of a feature that achieves the processing to be restricted.

9. An electronic endoscope system according to Claim 8, wherein the processing to be restricted is enlargement based on electronic zooming, and the presentation of an electronic zooming magnification is restricted.

10. An electronic endoscope system comprising:
a signal processing unit that converts a signal read from a solid-state imaging device incorporated in an electronic endoscope into a predetermined video signal;
a detecting means for detecting the connection to the signal processing unit; and
a restricting means for restricting processing to be performed by the signal processing unit according to the result of detecting performed by the detecting means.
